(19) **Europäisches Patentamt**

**European Patent Office**

**Office européen des brevets**

(11) **EP 0 815 833 B1**

(12) **EUROPEAN PATENT SPECIFICATION**

(45) Date of publication and mention
of the grant of the patent:
**09.04.2003  Bulletin 2003/15**

(51) Int Cl.7: **A61K 7/32**

(21) Application number: **97109895.9**

(22) Date of filing: **18.06.1997**

(54) **Malodour preventing agents**

Mittel zur Verhinderung von schlechten Gerüchen

Agents de prévention de mauvaises odeurs

(84) Designated Contracting States:
**CH DE ES FR GB IT LI NL**

(30) Priority: **24.06.1996  EP 96110149**

(43) Date of publication of application:
**07.01.1998  Bulletin 1998/02**

(73) Proprietor: **Givaudan SA**
**1214 Vernier-Genève (CH)**

(72) Inventors:
• **Acuna, Gonzalo**
**8953 Dietikon (CH)**

• **Frater, Georg**
**8400 Winterthur (CH)**
• **Gygax, Peter**
**8117 Fällanden (CH)**

(74) Representative: **Patentanwälte**
**Schaad, Balass, Menzl & Partner AG**
**Dufourstrasse 101**
**Postfach**
**8034 Zürich (CH)**

(56) References cited:
**EP-A- 0 036 134**     **EP-A- 0 126 483**
**US-A- 5 429 816**

## Description

The invention relates to agents preventing (the formation of) human malodour. In particular, the invention relates to the use of several classes of compounds which can act as such agents in cosmetic products, such as deodorants and antiperspirants. These compounds are normally odourless or nearly so, but upon contacting the skin as for example, in skin care compositions or in personal care compositions, they prevent malodour.

The present invention now provides compounds which show a low level of odour, or are even odourless, prior to application to the skin, and which prevent, supress (the formation of) or attenuate malodour after application to the skin, in particular to the skin in the axilla.

The compounds under consideration are compounds of the formula

$$X\text{-}(Z)_n\text{-}CO\text{-}NH\text{-}CH\text{-}(COO\text{-}Y)\text{-}CH_2CH_2CONH_2 \qquad\qquad I$$

wherein

| | |
|---|---|
| X = H, | alkyl, cycloalkyl, alkenyl, cycloalkenyl, aryl |
| | whereby each radical may also contain elements such as N, O, S, P, CO, and each of these radicals may be subsituted by groups, such as cycloalkyl, cycloalkenyl, aryl, hydroxy or acyloxy, e.g. alkanoyloxy, the radicals may further contain groups such as COOH, $COOR^1$, CN, $CONHR^2$ |
| Y= | H or X |
| Z= | O or S |
| $R^1$ | is alkyl, alkenyl, cycloalkyl |
| $R^2$ | is alkyl or substituted alkyl, e.g. $-CH(COOY)CH_2CH_2CONH_2$ |
| n= | 0,1 |
| | and,where, |
| n= | 0 |
| X | may also be $NH_2$, $NHR^1$, $NR^1_2$ |

and alkali, earth alkali, aluminium or, optionally substituted, ammonium salts of compounds, wherein Y = H.

Examples for the radicals X are:

Methyl,
ethyl,
propyl,
isopropyl,
tert.butyl,
sec.butyl,
isobutyl,
n-butyl,
pentyl,
hexyl,
heptyl,
octyl,
2-octyl,
nonyl,
2-nonyl,
decyl,
2-decyl,
undecyl,
2-undecyl,
dodecyl,
tridecyl,
tetradecyl,
pentadecyl,
hexadecyl,
heptadecyl,

octadecyl,
cyclopentyl,
cyclohexyl,
2-cyclohexylethyl,
2,6-dimethylheptyl,
geranyl,
neryl,
citronellyl,
9-decenyl,
2,6-dimethyl-5-heptenyl,
2,6-dimethyl-1,5-heptadienyl,
8,11-heptadecadienyl,
8-heptadecenyl,
cyclopentenyl,
cyclohexenyl,
phenyl,
p-methoxyphenyl,
benzyl,
2-phenylethyl,
1-phenylethyl,
2-(p-methoxyphenyl)-ethenyl,
3-(p-methylphenyl)-2-propyl,
3-(p-isopropylphenyl)-2-propyl,
3-(p-tert.butylphenyl)-2-propyl,
2,5,8-trioxanonyl,
acetonyl,
aminomethyl,
hydroxymethyl,
1-hydroxyethyl,
dimethylaminomethyl,
1-phenyl-1-aminoethyl,
carboxymethyl,
2-carboxyethyl,
3-carboxypropyl,
4-carboxybutyl,
5-carboxypentyl,
6-carboxyhexyl,
7-carboxyheptyl,
8-carboxyoctyl,
9-carboxynonyl,
10-carboxy-2,5,8-trioxanonyl,
7-carboxamido-5-carboxy-4-aza-3-oxo-heptyl,
8-carboxamido-6-carboxy-5-aza-4-oxo-octyl,
9-carboxamido-7-carboxy-6-aza-5-oxo-nonyl,
10-carboxamido-8-carboxy-7-aza-6-oxo-decyl,
11-carboxamido-9-carboxy-8-aza-7-oxo-undecyl,
14-carboxamido-12-carboxy-11-aza-10-oxo-tetradecyl,
2-pentyl-cyclopropyl,
menthyl,
terpineyl, etc.

The following known N-acylglutamines I are of particular importance:

N-formylglutamin
N-acetylglutamin
N-decanoylglutamin
N-undecanoylglutamin
N-dodecanoylglutamin

N-tetradecanoylglutamin
N-hexydecanoylglutamin
N-octadecanoylglutamin
N-phenylacetylglutamin
N-benzoylglutamin
N-carbonyloxymethylglutamin
N-carbonyloxyethylglutamin
N-tert.butoxycarbonylglutamin
N-carbonyloxybenzylglutamin
N-formylglutamin-benzylester
N-acetylglutamin-methylester
N-benzoylglutamin-methylester
N-carbonyloxybenzylglutamin-benzylester
N-acetylglutamin-aluminium-salt
N-carbamoylglutamin.

The following novel N-acylglutamines are also of particular importance:

N-2-methylheptanoylglutamin
N-10-undecenoylglutamin
N-3,7-dimethyl-6-octenoylglutamin
N-3,7-dimethyl-2,6-octadienoylglutamin
N-cinnamoylglutamin
N-3,6,9-trioxadecanoylglutamin
N-2-pentyl-cyclopropanoylglutamin
4-carbamoyl-2-(3-(3-carbamoyl-1-carboxypropylcarbamoyl)-propanoylamino)-butyric acid
4-carbamoyl-2-(4-(3-carbamoyl-1-carboxypropylcarbamoyl)-butanoylamino)-butyric acid
4-carbamoyl-2-(5-(3-carbamoyl-1-carboxypropylcarbamoyl)-pentanoylamino)-butyric acid
4-carbamoyl-2-(6-(3-carbamoyl-1-carboxypropylcarbamoyl)-hexanoylamino)-butyric acid
4-carbamoyl-2-(7-(3-carbamoyl-1-carboxypropylcarbamoyl)-heptanoylamino)-butyric acid
3-(3-carbamoyl-1-carboxypropylcarbamoyl)-propanoic acid
4-(3-carbamoyl-1-carboxypropylcarbamoyl)-butanoic acid
5-(3-carbamoyl-1-carboxypropylcarbamoyl)-pentanoic acid
6-(3-carbamoyl-1-carboxypropylcarbamoyl)-hexanoic acid
7-(3-carbamoyl-1-carboxypropylcarbamoyl)-heptanoic acid
4-carbamoyl-2-[2-(2-(2-[(3-carbamoyl-1-carboxypropylcarbamoyl)-methoxy]-ethoxy)-ethoxy)-acetylamino]-butyric acid
N-acetylglutamin-benzylester
N-acetylglutamin-dodecylester
N-acetylglutamin-tetradecylester
N-acetylglutamin-2-phenylethylester
N-acetylglutamin-geranylester
N-acetylglutamin-citronellylester
N-dodecanoylglutamin-2-phenylethylester
N-benzoylglutamin-phenylethylester
N-benzoylglutamin-benzylester
N-carbonyloxybenzylglutamin-geranylester
N-carbonyloxy-2-phenylethyl-glutamin-2-phenylethylester
N-acetylglutamin-sodium salt
N-acetylglutamin-potassium salt
N-dodecanoylglutamin-sodium salt
N-dodecanoylglutamin-potassium salt
N-dodecanoylglutamin-ammonium salt.

From this compilation it can be gathered, that a wide variety of compounds come into consideration. It can, for example, be seen that alkyl extends from $C_1$ to $C_{30}$ alkyl and alkenyl from $C_2$ to $C_{30}$ alkenyl, and one or more unsaturations may be present, and the respective chains may be linear or branched. Cycloalkyl and cycloalkenyl may extend from $C_3$ to $C_{12}$. Alkanoyloxy is preferably $C_{12\text{-}20}$-alkanoyloxy.

The aromatic rings encompass in particular, optionally substituted

one or more benzene rings
naphthalene.

The heterocycles encompass in particular, optionally substituted

pyridine
pyrrole
pyrrolidine
pyrimidine
furane
thiophene
tetrahydrofuran
quinoline
furanose
pyranose.

The compounds I - or any mixture of such compounds - may preferably be used to prevent, suppress or attenuate undesirable odours, in particular human malodour, e.g. axillary malodour which is the result of bacterial degradation of primarily odourless substances present in apocrine sweat. This form of sweat is produced by the apocrine glands present in the skin of the underarm. Bacteria from the underarm region degrade some of the compounds present in the apocrine sweat resulting in the release of volatile compounds responsible for the characteristic axilla sweat malodour.

The compounds I may preferably be used in consumer products, i.e. cosmetic products destined for application to human skin such as underarm deodorants or antiperspirants or other deodorants contacting the body, or in lotions, baby powders, baby lotions, ointments, foot products, body wipes, colognes, after-shave lotions, shaving creams, etc.

The compounds I are virtually odourless under normal temperature and atmospheric conditions, i.e. about 10-50 degrees Celsius and about 20 to 100% relative humidity.

The compounds I are not limited to any particular stereoisomers, all possible stereoisomers as well as racemates are thus included within the scope of formula I.

The compounds I permit the development of methods useful in - consumer products destined for attenuating or preventing human malodour. These compounds may be used individually in an amount effective to enhance this goal.

The amount required to produce the desired, overall effect varies depending upon the particular compounds I chosen, the product in which it will be used, and the particular effect desired.

For example, depending upon the selection and concentration of the compound chosen, when added I either singly or as a mixture e.g. to a deodorant composition at levels ranging from about 0.1 (or lower) to about 10% (or even higher) by weight, or most preferred about 0.25 to about 4% by weight formed composition serves to attenuate or to prevent the underarm odour, depending upon the selection and use levels of the compounds I.

The compounds I can accordingly be used in the manufacture of compositions used in the preparation of cosmetic products e.g. deodorants and antiperspirants, and as is evident from the above compilation, a broad range of known odorants or odorant mixtures can be used. In the manufacture of such compositions the known odorants or odorant mixtures set forth above can be used according to methods known to the perfumer, such as e.g. from W.A. Poucher, Perfumes, Cosmetics, Soaps, 2, 7th Edition, Chapman and Hall, London 1974.

To sum up, the present invention relates to:

1. A composition for application to human skin, containing a malodour preventing or malodour attenuating effective amount of at least one compound of the formula I in a cosmetically acceptable carrier.

2. A cosmetic product e.g. a personal body deodorant or antiperspirant article, containing at least one compound I.

3. A method of suppressing human body malodour by means of compounds of the formula I, which comprises the application to human skin of a cosmetic product as defined above.

4. The use of a composition as defined above in a cosmetic product e.g. a personal body deodorant or antiperspirant composition.

The compounds I can be prepared by using standard methods known to the skilled chemist. These standard methods can be found in the chemical literature.

N-acyl-glutamins or their esters can be prepared according to standard methods as described for example in Houben-Weyl, Methoden der Organischen Chemie, Synthese von Peptiden, Volume 15/1 p 46-305 (1974) by reaction of glutamin or glutamin esters with activated derivatives of carbonic or carboxylic acids. Activated derivatives are for example acid- or carbonic acid-halides, -anhydrides or activated esters such as N-hydroxybenzotriazinesters to name just one example. The base which may in some cases be indicated can be organic (Z. Grzonka, Synthesis 661 [1974]) or inorganic (O. Keller, Org. Synth. Coll. Vol. VII, 74 [1974] or P. Karrer, Helv. Chim. Acta 9, 301 [1926]). Depending on each individual case, the reactions are conveniently run in organic aprotic solvents, in organic protic solvents or in mixtures of organic solvents with water or even in water. The temperature is usually in the range of -40° to 150°C, preferably -20° to 120°C.

N-Acylglutaminesters can also be prepared from N-acylglutamin by esterification methods, preferably those used in peptide chemistry. An example is the reaction of an appropriate halide with acylglutamin in the presence of a base. This base is preferably diazabicycloundecene (DBU) as, e.g. described by N. Ono, Bull. Chem. Soc. Jap. 51, 2401 (1978). In this case, the solvent is preferably aprotic, examples are hydrocarbons, ethers, esters or N-disubstituted amides, etc. The reaction temperature is preferably between 0° and 100°C.

Convenient methods are outlined in the Examples.

Example 1

N-BOC-glutamin-benzylester

147 g N-BOC-glutamin (t-butyloxycarbonyl-glutamin), 77 g benzylchloride and 4 g benzylbromide were refluxed in 600 ml tetrahydrofuran for 2.5 hours in the presence of a 5% excess of diazabicycloundecene. After the removal of the solvent, the residue was taken up in water-ethylacetate and washed with citric acid, sodium bicarbonate and water. Evaporating of the solvent and recrystallization from ethylacetate-hexane gave 120 g white crystals. NMR (DMSO-d$_6$) among others: 7.36 (s,5H), 5.12 (dxd,2H), 3.989 (m,1H), 2.15 (t,2H), 1.38 (s,9H).

N-Lauroyl-glutamin (N-dodecanoyl-glutamin)

9.22 g lauroylchloride dissolved in 46 ml dioxane were slowly added to 6.72 g glutamine and 13.94 g triethylamin in 92 ml water and 92 ml dioxane, the temperature being 0°C. After stirring at room temperature for 1 hour, the dioxane was evaporated and the remaining solution was acidified to pH 1 with HCl. The crystals were filtered off and recrystallized from acetone and then from methanol: 9.9 g white needles. NMR (DMSO-d$_6$) among others: 8.05 (d,1H), 7.3 (s,1H), 6.78 (s,1H), 4.13 (m,1H), 2.03-2.18 (m,4H).

The following compounds were prepared according to the same procedure:

N-decanoyl-glutamin
N-tetradecanoyl-glutamin
N-hexadecanoyl-glutamin
N-octadecanoyl-glutamin
N-10-undecenoyl-glutamin
2-methylheptanoyl-glutamin.

Example 2

N-tert.butyloxycarbonylglutamin

150 g di-tert.butyldicarbonate in 300 ml dioxane were added slowly at 0°C to 100 g L-glutamin in 500 ml water and 207.5 g triethylamin. After stirring overnight, the organic solvents were removed and the residue was brought to pH 1 with conc. HCl. The aqueous phase was extracted several times with ethylacetate and the combined organic phases were then washed with water and brine before being evaporated. 147 g BOC-glutamine were obtained as a colourless glass.

N-benzyloxycarbonylglutamin was prepared according to the same procedure but using dibenzyl-dicarbonate as the reagent.

Example 3

The following sets forth examples for the use of the Compounds I in various products. The methods of forming the following compositions are well known to those skilled in the art. All formulations may contain additional ingredients known to those skilled in the art, e.g. colourants, opacifiers, buffers, antioxidants, vitamins, emulsifiers, UV absorbers, silicones and the like. All products can also be buffered to the desired pH. All values are % w/w.

Deo-colognes (four exemplary compositions):

| Compound (I) | 0.5 | 1.5 | 2.5 | 6.0 |
|---|---|---|---|---|
| Fragrance | 0.5 | 1.5 | 2.5 | 6.0 |
| Triclosan (Ciba-Geigy) | 1.0 | - | 0.75 | 1.0 |
| Ethanol | 100 | 100 | 100 | 100 |

Deo-Sticks:

| Antiperspirant stick | |
|---|---|
| Ethylene Glycol Monostearate | 7.0 |
| Shea butter | 3.0 |
| Neobee 1053 (PVO International) | 12.0 |
| Generol 122 (Henkel) | 5.0 |
| Kesscowax B (Akzo) | 17.0 |
| Dimethicone Dow Corning 345 | 35.0 |
| Aluminium Sesquichlorhydrate | 20.0 |
| Compound (I) | 0.5 |
| Fragrance | 0.5 |

| Antiperspirant stick | |
|---|---|
| Steary Alcohol | 17.0 |
| Castor Wax | 3.0 |
| Talc | 5.0 |
| Aluminum Zirconium Tetrachlorhydrate | 20.0 |
| Compound (I) | 1.0 |
| Fragrance | 1.0 |
| Dimethicone Dow 245 | to 100.0 |

| Clear Deodorant Stick | |
|---|---|
| Witconol APM | 43.0 |
| Propylene Glycol | 20.0 |
| Alcohol 39C | 20.0 |
| Water | 7.0 |
| Monamid 150ADD | 5.0 |
| Millithix 925 | 2.0 |
| Ottasept Extra | 0.5 |
| Compound (I) | 0.75 |
| Fragrance | 0.75 |

| Deodorant Stick | |
|---|---|
| Propylene Glycol | 69.0 |
| Water | 21.8 |
| Triclosan | 0.2 |
| Sodium Stearate | 8.0 |
| Compound (I) | 0.5 |
| Fragrance | 0.5 |

| Alcohol free Deodorant Stick | |
|---|---|
| Propylene Glycol-3 Myristyl Ether (Witconol APM) | 36.0 |
| Propylene Glycol | 36.0 |
| Water | 19.0 |
| Triclosan | 0.25 |
| Sodium Stearate | 7.75 |
| Compound (I) | 0.5 |
| Fragrance | 0.5 |

| Antiperspirant Aerosol | |
|---|---|
| Absolute Ethanol | 15.0 |
| Zirconium Aluminum tetrachlorhydrate | 5.0 |
| Bentone 38 | 1.5 |
| Compound (I) | 0.75 |
| Fragrance | 0.75 |
| S-31 Hydrocarbon propellant | to 100 |

| Antiperspirant Pump | |
|---|---|
| Water | 57.5 |
| Aluminum Sesquichlorhydrate | 20.0 |
| Triton X-102 (Union Carbide) | 2.0 |
| Dimethyl Isosorbide (ICI) | 20.0 |
| Compound (I) | 0.25 |
| Fragrance | 0.25 |

| Roll-On | |
|---|---|
| Dimethicone DC 354 (Dow Corning) | 69.0 |
| Bentone 38 | 10.0 |
| Rezal 36 GP (Reheis Chem.Co.) | 20.0 |
| Compound (I) | 0.5 |
| Fragrance | 0.5 |

In the above, the following components were used:

| Triclosan | 5-chloro-2-(2,4-dichlorophenoxy) phenol |
|---|---|
| Neobee 1053 | glycerol tricaprate/caprylate |
| Generol 122 | soya sterol |
| Kesscowax B | cetyl alcohol and glycol polymer |

(continued)

| | |
|---|---|
| Witconol APM | polypropylene glycol-3 myristyl ether |
| Monamid 150ADD | cocoamide diethanolamine |
| Millithix 925 | dibenzylidene sorbitol |
| Ottasept Extra | quaternium 18 hectorite |
| Bentone 38 | quaternium 18 hectorite |
| Triton X-102 | octoxynol-13 |
| Dimethicone DC 354 | mixture of fully methylated linear siloxane polymers end-blocked with trimethylsiloxy units |
| Rezal 36 GP | aluminium zirconium tetrachlorohydrexglycine |

**Claims**

1. A composition for application to human skin, containing an amount effective to prevent, suppress or attenuate human malodour of at least one compound of the formula

$$X-(Z)_n-CO-NH-CH-(COO-Y)-CH_2CH_2CONH_2 \text{ I} \hspace{2cm} I$$

wherein

| | |
|---|---|
| X = | H alkyl, cycloalkyl, alkenyl, cycloalkenyl, aryl |
| | each radical may also contain elements such as N, O, S, P, CO, whereby each of these radicals may be subsituted by groups, such as cycloalkyl, cycloalkenyl, aryl, hydroxy or acyloxy, the radicals may further contain groups such as COOH, $COOR^1$, CN, $CONHR^2$ |
| Y= | H or X |
| Z= | O or S |
| $R^1$ | is alkyl, alkenyl, cycloalkyl |
| $R^2$ | is alkyl or substituted alkyl |
| n= | 0,1 |
| | and, where, |
| n= | 0 |
| X | may also be $NH_2$, $NHR^1$, $NR^1_2$ |

and alkali, earth alkali, aluminium or, optionally substituted, ammonium salts of compounds, wherein Y = H in a cosmetically acceptable carrier.

2. A composition according to claim 1 selected from the group consisting of deodorants, antiperspirants, underarm deodorants or antiperspirants, lotions, baby powders, baby lotions, ointments, foot products, body wipes, colognes, and shaving creams.

3. A composition according to Claim 1, wherein the compound of formula I is selected from the group consisting of

N-formylglutamin
N-acetylglutamin
N-decanoylglutamin
N-undecanoylglutamin
N-dodecanoylglutamin
N-tetradecanoylglutamin
N-hexydecanoylglutamin
N-octadecanoylglutamin
N-phenylacetylglutamin
N-benzoylglutamin
N-carbonyloxymethylglutamin
N-carbonyloxyethylglutamin
N-tert.butoxycarbonylglutamin

N-carbonyloxybenzylglutamin
N-formylglutamin-benzylester
N-acetylglutamin-methylester
N-benzoylglutamin-methylester
N-carbonyloxybenzylglutamin-benzylester
N-acetylglutamin-aluminium-salt
N-2-methylheptanoylglutamin
N-10-undecenoylglutamin
N-3,7-dimethyl-6-octenoylglutamin
N-3,7-dimethyl-2,6-octadienoylglutamin
N-cinnamoylglutamin
N-3,6,9-trioxadecanoylglutamin
N-2-pentyl-cyclopropanoylglutamin
N-benzyloxy-carbonylglutamin
N-tert.butyloxycarbonylglutamin-benzyl ester
4-carbamoyl-2-(3-(3-carbamoyl-1-carboxypropylcarbamoyl)-propanoylamino)-butyric acid
4-carbamoyl-2-(4-(3-carbamoyl-1-carboxypropylcarbamoyl)-butanoylamino)-butyric acid
4-carbamoyl-2-(5-(3-carbamoyl-1-carboxypropylcarbamoyl)-pentanoylamino)-butyric acid
4-carbamoyl-2-(6-(3-carbamoyl-1-carboxypropylcarbamoyl)-hexanoylamino)-butyric acid
4-carbamoyl-2-(7-(3-carbamoyl-1-carboxypropylcarbamoyl)-heptanoylamino)-butyric acid
3-(3-carbamoyl-1-carboxypropylcarbamoyl)-propanoic acid
4-(3-carbamoyl-1-carboxypropylcarbamoyl)-butanoic acid
5-(3-carbamoyl-1-carboxypropylcarbamoyl)-pentanoic acid
6-(3-carbamoyl-1-carboxypropylcarbamoyl)-hexanoic acid
7-(3-carbamoyl-1-carboxypropylcarbamoyl)-heptanoic acid
4-carbamoyl-2-[2-(2-(2-[(3-carbamoyl-1-carboxypropylcarbamoyl)-methoxy]-ethoxy)-ethoxy)-acetylamino]-butyric acid
N-acetylglutamin-benzylester
N-acetylglutamin-dodecylester
N-acetylglutamin-tetradecylester
N-acetylglutamin-2-phenylethylester
N-acetylglutamin-geranylester
N-acetylglutamin-citronellylester
N-dodecanoylglutamin-2-phenylethylester
N-benzoylglutamin-phenylethylester
N-benzoylglutamin-benzylester
N-carbonyloxybenzylglutamin-geranylester
N-carbonyloxy-2-phenylethyl-glutamin-2-phenylethylester
N-acetylglutamin-sodium and -potassium salt
N-dodecanoylglutamin-sodium, -potassium and -ammonium salt
N-carbamoylglutamin.

4.  A personal body deodorant containing at least one compound I as defined in Claim 1.

5.  An antiperspirant containing at least one compound I as defined in claim 1.

6.  A compound of formula I as defined in Claim 1 and selected from the group consisting of

N-2-methylheptanoylglutamin
N-10-undecenoylglutamin
N-3,7-dimethyl-6-octenoylglutamin
N-3,7-dimethyl-2,6-octadienoylglutamin
N-cinnamoylglutamin
N-3,6,9-trioxadecanoylglutamin
N-2-pentyl-cyclopropanoylglutamin
4-carbamoyl-2-(3-(3-carbamoyl-1-carboxypropylcarbamoyl)-propanoylamino)-butyric acid
4-carbamoyl-2-(4-(3-carbamoyl-1-carboxypropylcarbamoyl)-butanoylamino)-butyric acid
4-carbamoyl-2-(5-(3-carbamoyl-1-carboxypropylcarbamoyl)-pentanoylamino)-butyric acid

4-carbamoyl-2-(6-(3-carbamoyl-1-carboxypropylcarbamoyl)-hexanoylamino)-butyric acid
4-carbamoyl-2-(7-(3-carbamoyl-1-carboxypropylcarbamoyl)-heptanoylamino)-butyric acid
3-(3-carbamoyl-1-carboxypropylcarbamoyl)-propanoic acid
4-(3-carbamoyl-1-carboxypropylcarbamoyl)-butanoic acid
5-(3-carbamoyl-1-carboxypropylcarbamoyl)-pentanoic acid
6-(3-carbamoyl-1-carboxypropylcarbamoyl)-hexanoic acid
7-(3-carbamoyl-1-carboxypropylcarbamoyl)-heptanoic acid
4-carbamoyl-2-[2-(2-(2-[(3-carbamoyl-1-carboxypropylcarbamoyl)methoxy]-ethoxy)-ethoxy)-acetylamino]-butyric acid
N-acetylglutamin-benzylester
N-acetylglutamin-dodecylester
N-acetylglutamin-tetradecylester
N-acetylglutamin-2-phenylethylester
N-acetylglutamin-geranylester
N-acetylglutamin-citronellylester
N-dodecanoylglutamin-2-phenylethylester
N-benzoylglutamin-phenylethylester
N-benzoylglutamin-benzylester
N-carbonyloxybenzylglutamin-geranylester
N-carbonyloxy-2-phenylethyl-glutamin-2-phenylethylester
N-acetylglutamin-sodium and -potassium salt
N-dodecanoylglutamin-sodium, -potassium and -ammonium salt.

**7.** A method of preventing, suppressing or attenuating human body malodour by means of compounds of the formula I as defined in Claim 1, which comprises the application to human skin of a cosmetic product as defined in Claims, 4 or 5.

**8.** The use of a compound I as defined in Claim 1 in a composition for application to human skin in a personal body deodorant.

**9.** The use of a compound I as defined in Claim 1 in a composition for application to human skin in an antiperspirant composition.

**Patentansprüche**

**1.** Eine Zusammensetzung für die Anwendung auf menschlicher Haut, welche eine zur Vorbeugung, Unterdrückung oder Verminderung von menschlichem Schweiss wirksame Menge mindestens einer Verbindung der Formel I enthält,

$$X-(Z)_n-CO-NH-CH-(COO-Y)-CH_2CH_2CONH_2 \qquad\qquad I$$

in der

X = H, Alkyl, Cycloalkyl, Alkenyl, Cycloalkenyl, Aryl ist, wobei jeder Rest ebenfalls Elemente wie N,O,S,P,CO enthalten kann, wobei jeder dieser Reste durch Gruppen wie Cycloalkyl, Cycloalkenyl, Aryl, Hydroxy oder Acyloxy substituiert sein kann, die Reste überdies Gruppen wie COOH, COOR$^1$, CN, CONHR$^2$ enthalten können,
Y = H oder X
Z = O oder S
R$^1$ Alkyl, Alkenyl, Cycloalkyl
R$^2$ Alkyl oder substituiertes Alkyl
n = 0, 1 ist,
und wenn
n = 0
X NH$_2$, NHR$^1$, NR$^1_2$ sein kann,

und Alkali-, Erdalkali-, Aluminium- oder wahlweise substituierte Ammoniumsalze der Vebindungen, in denen Y = H ist,
in einem kosmetisch annehmbaren Träger.

2.  Eine Zusammensetzung nach Anspruch 1, ausgewählt aus der Gruppe bestehend aus Deodorantien, Antiperspirantien, Unterarm-Deodorantien oder Antiperspirantien, Lotionen, Babypudern, Babylotionen, Salben, Fussprodukten, Körperwischtüchern, Rasierwassern und Rasiercremes.

3.  Eine Zusammensetzung nach Anspruch 1, in der die Verbindung der Formel I ausgewählt ist aus der Gruppe bestehend aus

    N-Formylglutamin

    N-Acetylglutamin

    N-Decanoylglutamin

    N-Undecanoylglutamin

    N-Dodecanoylglutamin

    N-Tetradecanoylglutamin

    N-Hexydecanoylglutamin

    N-Octadecanoylglutamin

    N-Phenylacetylglutamin

    N-Benzoylglutamin

    N-Carbonyloxymethylglutamin

    N-Carbonyloxyethylglutamin

    N-tert. Butoxycarbonylglutamin

    N-Carbonyloxybenzylglutamin

    N-Formylglutamin-benzylester

    N-Acetylglutamin-methylester

    N-Benzoylglutamin-methylester

    N-Carbonyloxybenzylglutamin-benzylester

    N-Acetylglutamin-Aluminiumsalz

    N-2-Methylheptanoylglutamin

    N-10-Undecenoylglutamin

    N-3,7-Dimethyl-6-octenoylglutamin

    N-3,7-Dimethyl-2,6-octadienoylglutamin

    N-Cinnamoylglutamin

N-3,6,9-Trioxadecanoylglutamin

N-2-Pentyl-cyclopropanoylglutamin

N-Benzyloxy-carbonylglutamin

N-tert. Butyloxycarbonylglutaminbenzylester

4-Carbamoyl-2-(3-(3-carbamoyl-1-carboxypropylcarbamoyl)-propanoyl-amino)-buttersäure

4-Carbamoyl-2-(4-(3-carbamoyl-1-carboxypropylcarbamoyl)-butanoyl-amino)-buttersäure

4-Carbamoyl-2-(5-(3-carbamoyl-1-carboxypropylcarbamoyl)-pentanoyl-amino)-buttersäure

4-Carbamoyl-2-(6-(3-carbamoyl-1-carboxypropylcarbamoyl)-hexanoyl-amino)-buttersäure

4-Carbamoyl-2-(7-(3-carbamoyl-1-carboxypropylcarbamoyl)-heptanoyl-amino)-buttersäure

3-(3-Carbamoyl-1-carboxypropylcarbamoyl)-propansäure

4-(3-Carbamoyl-1-carboxypropylcarbamoyl)-butansäure

5-(3-Carbamoyl-1-carboxypropylcarbamoyl)-pentansäure

6-(3-Carbamoyl-1-carboxypropylcarbamoyl)-hexansäure

7-(3-Carbamoyl-1-carboxypropylcarbamoyl)-heptansäure

4-Carbamoyl-2-[2-(2-(2-[(3-carbamoyl-1-carboxypropyl]carbamoyl)-methoxy]-ethoxy)-ethoxy)-acetylamino]-buttersäure

N-Acetylglutamin-benzylester

N-Acetylglutamin-dodecylester

N-Acetylglutamin-tetradecylester

N-Acetylglutamin-2-phenylethylester

N-Acetylglutamin-geranylester

N-Acetylglutamin-citronellylester

N-Dodecanoylglutamin-2-phenylethylester

N-Benzoylglutamin-phenylethylester

N-Benzoylglutamin-benzylester

N-Carbonyloxybenzylglutamin-geranylester

N-Carbonyloxy-2-phenylethylglutamin-2-phenylethylester

N-Acetylglutamin-Natrium- und Kaliumsalz

N-Dodecanoylglutamin-Natrium-, Kalium- und Ammoniumsalz

N-Carbamoylglutamin

**EP 0 815 833 B1**

4. Persönlicher Körperdeodorant beinhaltend wenigstens eine Verbindung I wie in Anspruch 1 definiert.

5. Ein Antiperspirans beinhaltend wenigstens eine Verbindung I wie in Anspruch 1 definiert.

6. Eine Verbindung der Formel I wie in Anspruch 1 definiert und ausgewählt aus der Gruppe bestehend aus

N-2-Methylheptanoylglutamin

N-10-Undecenoylglutamin

N-3,7-Dimethyl-6-octenoylglutamin

N-3,7-Dimethyl-2,6-octadienoylglutamin

N-Cinnamoylglutamin

N-3,6,9-Trioxadecanoylglutamin

N-2-Pentyl-cyclopropanoylglutamin

4-Carbamoyl-2-(3-(3-carbamoyl-1-carboxypropylcarbamoyl)-propanoyl-amino)-buttersäure

4-Carbamoyl-2-(4-(3-carbamoyl-1-carboxypropylcarbamoyl)-butanoyl-amino)-buttersäure

4-Carbamoyl-2-(5-(3-carbamoyl-1-carboxypropylcarbamoyl)-pentanoyl-amino)-buttersäure

4-Carbamoyl-2-(6-(3-carbamoyl-1-carboxypropylcarbamoyl)-hexanoyl-amino)-buttersäure

4-Carbamoyl-2-(7-(3-carbamoyl-1-carboxypropylcarbamoyl)-heptanoyl-amino)-buttersäure

3-(3-Carbamoyl-1-carboxypropylcarbamoyl)-propansäure

4-(3-Carbamoyl-1-carboxypropylcarbamoyl)-butansäure

5-(3-Carbamoyl-1-carboxypropylcarbamoyl)-pentansäure

6-(3-Carbamoyl-1-carboxypropylcarbamoyl)-hexansäure

7-(3-Carbamoyl-1-carboxypropylcarbamoyl)-heptansäure

4-Carbamoyl-2-[2-(2-(2-[(3-carbamoyl-1-carboxypropyl]carbamoyl)-methoxy]-ethoxy)-ethoxy)-acetylamino]-buttersäure

N-Acetylglutamin-benzylester

N-Acetylglutamin-dodecylester

N-Acetylglutamin-tetradecylester

N-Acetylglutamin-2-phenylethylester

N-Acetylglutamin-geranylester

N-Acetylglutamin-citronellylester

N-Dodecanoylglutamin-2-phenylethylester

N-Benzoylglutamin-phenylethylester

14

N-Benzoylglutamin-benzylester

N-Carbonyloxybenzylglutamin-geranylester

N-Carbonyloxy-2-phenylethylglutamin-2-phenylethylester

N-Acetylglutamin-Natrium- und Kaliumsalz

N-Dodecanoylglutamin-Natrium-, Kalium- und Ammoniumsalz.

7. Verfahren zur Vorbeugung, Unterdrückung oder Verminderung von menschlichem Schweiss durch Verbindungen der Formel I wie in Anspruch 1 definiert, welches die Anwendung eines kosmetischen Produktes wie in den Ansprüchen 4 oder 5 definiert auf menschlicher Haut beinhaltet.

8. Die Verwendung einer Verbindung I wie in Anspruch 1 definiert in einer Zusammensetzung zur Anwendung auf menschlicher Haut in einem persönlichen Körperdeodorant.

9. Die Verwendung einer Verbindung I wie in Anspruch 1 definiert in einer Zusammensetzung zur Anwendung auf menschlicher Haut in einer Antiperspirans-Zusammensetzung.


**Revendications**

1. Composition pour une application sur la peau humaine, contenant une quantité efficace afin de prévenir, supprimer ou atténuer les mauvaises odeurs d'origine humaine d'au moins un composé de formule

$$X\text{-}(Z)_n\text{-}CO\text{-}NH\text{-}CH\text{-}(COO\text{-}Y)\text{-}CH_2CH_2CONH_2$$

dans laquelle

X = H, un alkyle, cycloalkyle, alcényle, cycloalcényle, aryle
chaque radical peut également contenir des éléments, tels que N, O, S, P, CO, moyennant quoi chacun de ces radicaux peut être remplacé par des groupes, tels que cycloalkyle, cycloalcényle, aryle, hydroxy ou acyloxy, les radicaux peuvent également contenir des groupes, tels que COOH, COOR$^1$, CN, CONHR$^2$

| | |
|---|---|
| Y = | H ou X |
| Z = | O ou S |
| R$^1$ | est un alkyle, alcényle, cycloalkyle |
| R$^2$ | est un alkyle ou un alkyle substitué |
| n = | 0, 1 |
| et, où | n = O |
| X = | peut également être $NH_2$, $NHR^1$, $NR_2^1$ |

et les sels d'un alcali, d'un alcali terreux, d'aluminium ou d'ammonium facultativement substitué de composés, où Y = H
dans un porteur acceptable en cosmétique.

2. Composition selon la revendication 1, sélectionnée parmi le groupe constitué de déodorants, d'anti-transpirants, de déodorant ou d'anti-transpirants pour aisselles, de lotions, de poudres pour bébés, de lotions pour bébés, de pommades, de produits pour pieds, de lingettes pour le corps, d'eau de cologne et de crèmes à raser.

3. Composition selon la revendication 1, dans laquelle le composé de formule 1 est sélectionné parmi le groupe constitué de

N-formylgultamine
N-acétylglutamine
N-décanoylglutamine

N-undécanoylglutamine
N-dodécanolglutamine
N-tétradécanoylglutamine
N-hexy décanoylglutamine
N-octadécanoylglutamine
N-phénylacétylglutamine
N-benzoylglutamine
N-carbonyloxyméthylglutamine
N-carbonyloxyéthylglutamine
N-tert.butoxycarbonylglutamine
N-carbonyloxybenzylglutamine
N-formylglutamine-benzylester
N-acétylglutamine-méthylester
N-benzoylglutamine-méthylester
N-carbonyloxybenzylglutamine-benzylester sel d'aluminium de N-acétylglutamine
N-2-méthylheptanoylglutamine
N-10-undécénoylglutamine
N-3,7-diméthyl-6-octénoylglutamine
N-3,7-diméthyl-2,6-octadiénoylglutamine
N-cinnamoylglutamine
N-3,6,9-trioxadécanoylglutamine
N-2-pentyl-cyclopropanoylglutamine
N-benzyloxy-carbonylglutamine
N-tert.butyloxycarbonylglutamine-benzylester
acide 4-carbamoyl-2-(3-(3-carbamoyl-1-carboxypropylcarbamoyl)-propanoyl-amino)-butyrique
acide 4-carbamoyl-2-(4-(3-carbamoyl-1-carboxypropylcarbamoyl)-butanoyl-amino)-butyrique
acide 4-carbamoyl-2-(5-(3-carbamoyl-1-carboxypropylcarbamoyl)-pentanoyl-amino)-butyrique
acide 4-carbamoyl-2-(6-(3-carbamoyl-1-carboxypropylcarbamoyl)-hexanoyl-amino)-butyrique
acide 4-carbamoyl-2-(7-(3-carbamoyl-1-carboxypropylcarbamoyl)-heptanoyl-amino)-butyrique
acide 3-(3-carbamoyl-1-carboxypropylcarbamoyl)-propanoïque
acide 4-(3-carbamoyl-1-carboxypropylcarbamoyl)-butanoïque
acide 5-(3-carbamoyl-1-carboxypropylcarbamoyl)-pentanoïque
acide 6-(3-carbamoyl-1-carboxypropylcarbamoyl)-hexanoïque
acide 7-(3-carbamoyl-1-carboxypropylcarbamoyl)-heptanoïque
acide 4-carbamoyl-2-[2-(2-(2-[(3-carbamoyl-1-carboxypropylcarbamoyl)-méthoxy]-éthoxy)-éthoxy)-acétylamino]-butyrique
N-acétylglutamine-benzylester
N-acétylglutamine-dodécylester
N-acétylglutamine-tétradécylester
N-acétylglutamine-2-phényléthylester
N-acétylglutamin-géranylester
N-acétylglutamine-citronellylester
N-dodécanoylglutamine-2-phényléthylester
N-benzoylglutamine-phényléthylester
N-benzoylglutamine-benzylester
N-carbonyloxybenzylglutamine-géranylester
N-carbonyloxy-2-phényléthyl-glutamine-2-phényléthylester
sel de N-acétyl glutamine-sodium et -potassium
sel de N-dodécanoylglutamine-sodium, -potassium et -ammonium
N-carbamoylglutamine.

**4.** Déodorant personnel pour le corps contenant au moins un composé I, tel que défini dans la revendication 1.

**5.** Anti-transpirant contenant au moins un composé I, tel que défini dans la revendication 1.

**6.** Composé de formule I selon la revendication 1 et sélectionné parmi le groupe constitué de :

N-2-méthylheptanoylglutamine

N-10-undécénoylglutamine

N-3,7-diméthyl-6-octénoylglutamine

N-3,7-diméthyl-2,6-octadiénoylglutamine

N-cinnamoylglutamine

N-3,6,9-trioxadécanoylglutamine

N-2-pentyl-cyclopropanoylglutamine

acide 4-carbamoyl-2-(3-(3-carbamoyl-1-carboxypropylcarbamoyl)-propanoyl-amino)-butyrique

acide 4-carbamoyl-2-(4-(3-carbamoyl-1-carboxypropylcarbamoyl)-butanoyl-amino)-butyrique

acide 4-carbamoyl-2-(5-(3-carbamoyl-1-carboxypropylcarbamoyl)-pentanoyl-amino)-butyrique

acide 4-carbamoyl-2-(6-(3-carbamoyl-1-carboxypropylcarbamoyl)-hexanoyl-amino)-butyrique

acide 4-carbamoyl-2-(7-(3-carbamoyl-1-carboxypropylcarbamoyl)-heptanoyl-amino)-butyrique

acide 3-(3-carbamoyl-1-carboxypropylcarbamoyl)-propanoïque

acide 4-(3-carbamoyl-1-carboxypropylcarbamoyl)-butanoïque

acide 5-(3-carbamoyl-1-carboxypropylcarbamoyl)-pentanoïque

acide 6-(3-carbamoyl-1-carboxypropylcarbamoyl)-hexanoïque

acide 7-(3-carbamoyl-1-carboxypropylcarbamoyl)-heptanoïque

acide 4-carbamoyl-2-[2-(2-(2-[(3-carbamoyl-1-carboxypropylcarbamoyl)-méthoxy]-éthoxy)-éthoxy)-acétylamino]-butyrique

N-acétylglutamine-benzylester

N-acétylglutamine-dodécylester

N-acétylglutamine-tétradécylester

N-acétylglutamine-2-phényléthylester

N-acétylglutamin-géranylester

N-acétylglutamine-citronellylester

N-dodécanoylglutamine-2-phényléthylester

N-benzoylglutamine-phényléthylester

N-benzoylglutamine-benzylester

N-carbonyloxybenzylglutamine-géranylester

N-carbonyloxy-2-phényléthyl-glutamine-2-phényléthylester

sel de N-acétyl glutamine-sodium et -potassium

sel de N-dodécanoylglutamine-sodium, -potassium et -ammonium.

**7.** Procédé pour prévenir, supprimer ou atténuer les mauvaises odeurs du corps humain au moyen de composés de formule I selon la revendication 1, qui comprend l'application sur la peau humaine d'un produit cosmétique selon les revendications 4 ou 5.

**8.** Utilisation d'un composé I selon la revendication 1 dans une composition pour application sur la peau humaine dans un déodorant personnel pour le corps.

**9.** Utilisation d'un composé I selon la revendication 1 dans une composition pour application sur la peau humaine dans une composition anti-transpirante.